# EUROPEAN PATENT APPLICATION

(11) **EP 1 709 917 A1**
(43) Date of publication of application: **11.10.2006**
(21) Application number: 05425202.8
(22) Date of filing: 07.04.2005
(51) Int. Cl.: A61B 17/34

(54) **Retractable-tip needle for spinal anaesthesia**

(71) Applicant: Policare S.r.l., 52022 Cavriglia (IT)
(72) Inventor: Di Donato, Attilio, 03039 Sora (IT); Forino, Luciano, 10100 Torino (IT)
(74) Representative: D'Angelo, Fabio

(57) **Abstract**

There is described a needle (11) for spinal anaesthesia, having an outer body (12) for conducting anaesthetic into a patient's body through an opening (21); and a spindle (13) selectively movable inside the outer body (12) to seal/unseal the opening (21); the opening (21) being coaxial with an axis (B) of the needle (11).

## Description

The present invention relates to a needle for spinal or subarachnoid anaesthesia.

As is known, spinal anaesthesia is normally performed by injecting an anaesthetic into the spinal fluid, i.e. into the subarachnoid space between a first protective membrane of the spinal cord known as the dura mater, and a second protective membrane known as the pia mater and closer to the spinal cord than the dura mater.

More specifically, anaesthetic is injected into the spinal fluid by means of a needle comprising a tubular outer body for conducting the anaesthetic into the spinal fluid; and a spindle housed hermetically inside the outer body, and which, in use, is withdrawn from the outer body, in a direction parallel to the needle axis, to permit injection of the anaesthetic.

More specifically, at one axial end, the outer body has an elongated front opening located laterally with respect to, and extending parallel to, the needle axis, and for injecting the anaesthetic into the spinal fluid.

At the opposite end to the front opening, the outer body also has a rear hole coaxial with the needle, and which is sealed by the spindle when inserting the needle into the patient, and is opened by withdrawing the spindle, once the needle is inserted, to permit connection to a syringe containing the anaesthetic for injection.

Anaesthesia is performed by inserting the needle axially until a click indicates the needle has penetrated through the dura mater into the spinal fluid.

At this point, the spindle is withdrawn from the outer body, and the syringe is connected to the rear hole in the outer body to inject the anaesthetic through the front hole into the spinal fluid.

Known needles fail to provide for injecting the anaesthetic easily and effectively into the spinal fluid.

More specifically, because of the elongated shape and the lateral location of the front opening with respect to the needle axis, the click may occur when only a portion of the front opening is positioned inside the spinal fluid, while the rest of the front opening is positioned inside the dura mater. As a result, the anaesthetic is only injected partly into the spinal fluid, thus reducing its effectiveness.

Moreover, using known needles, the anaesthetic is injected unevenly into the subarachnoid space, in preferential directions corresponding to the radial directions out of the front opening.

Known needles fail to provide for injecting anaesthetic easily into regions, such as the cervical region, in which the subarachnoid space is small crosswise to the needle insertion direction.

Insertion of the needle into such regions, in fact, may result in injury caused by the front-opening end of the needle contacting the pia mater.

Finally, catheters, e.g. for continuous spinal anaesthesia, are practically impossible to insert inside known spinal anaesthesia needles, on account of the catheter requiring a first portion inserted inside the outer body coaxially with the needle, and a second portion for injection via the front opening and crosswise to the first portion.

It is an object of the present invention to provide a spinal anaesthesia needle designed to provide a straightforward, low-cost solution to the aforementioned drawbacks typically associated with known spinal anaesthesia needles.

According to the present invention, there is provided a needle for spinal anaesthesia, as claimed in Claim 1.

A preferred, non-limiting embodiment of the present invention will be described by way of example with reference to the accompanying drawings, in which:
Figure 1 shows a section, with parts removed for clarity, of an end portion of a known spinal anaesthesia needle in the operating condition;
Figure 2 shows a section, with parts removed for clarity, of an end portion of a spinal anaesthesia needle in accordance with the present invention in the operating condition;
Figure 3 shows a smaller-scale side view of the Figure 2 needle;
Figure 4 shows a smaller-scale exploded view of the Figure 2 needle;
Figures 5 to 8 show smaller-scale views of successive steps in the insertion of the Figure 2 needle.

With reference to the accompanying drawings, numbers 1 and 11 indicate, respectively, a known needle and a needle in accordance with the present invention, for injecting a spinal anaesthetic into the spinal fluid 5, i.e. into a region between a first protective membrane of the spinal cord known as the dura mater and indicated 6, and a second protective membrane of the spinal cord known as the pia mater (not shown) and located closer to the spinal cord than the dura mater 6.

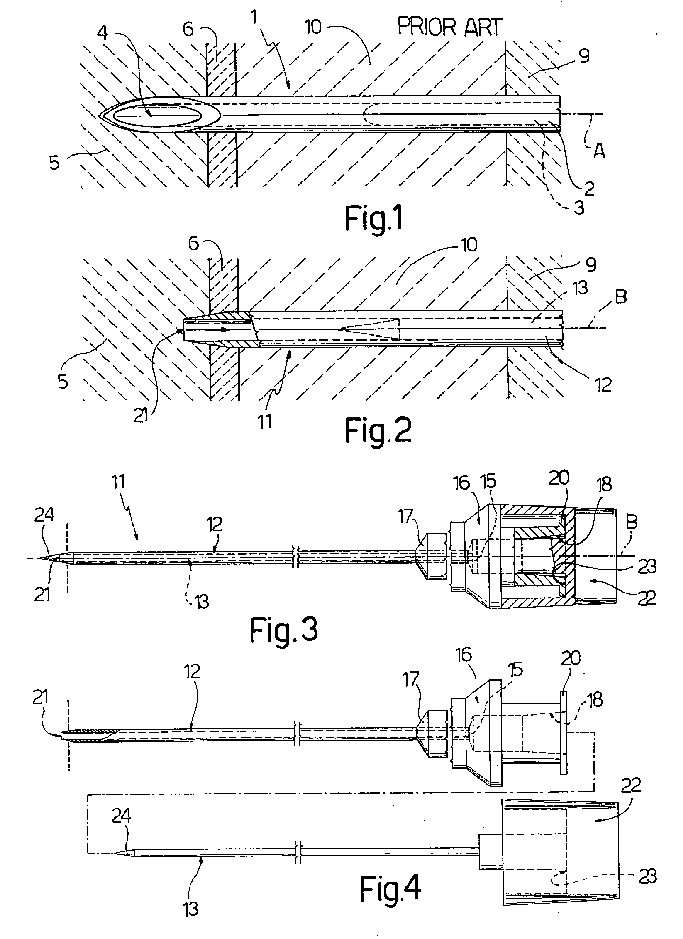

More specifically, to inject the anaesthetic into spinal fluid 5, needles 1 and 11 are inserted through a number of tissues (Figures 1, 2, 5-8) arranged successively from the epidermis to the dura mater 6 and known as the superspinous ligament 7, interspinous ligament 8, yellow ligament 9, and peridural space 10.

With particular reference to Figure 1, needle 1 extends along an axis A, and comprises a tubular outer body 2 coaxial with axis A and for conducting anaesthetic into spinal fluid 5; a spindle 3 inserted hermetically and coaxially inside outer body 2 and withdrawable, parallel to axis A, from the outer body; and an inserter (not shown in Figure 1) coaxially surrounding outer body 2 and for assisting insertion and correct positioning of needle 1 through the epidermis and the various tissues mentioned above.

More specifically, at one axial end, outer body 2 has a substantially oval, cutting or non-cutting, front opening 4 extending parallel, and laterally with respect, to axis A. Opening 4 is sealed by spindle 3 when inserting needle 1, and injects the anaesthetic into spinal fluid 5 once spindle 3 is withdrawn.

At the opposite axial end to opening 4, outer body 2 has a rear hole (not shown in Figure 1), which is sealed by spindle 3 when inserting needle 1, and which is opened, when spindle 3 is withdrawn, for hydraulic connection to a syringe (not shown in Figure 1) containing the anaesthetic.

Needle 1 is inserted successively through superspinous ligament 7, interspinous ligament 8, yellow ligament 9, peridural space 10, and dura mater 6 into spinal fluid 5.

The correct position of needle 1, with opening 4 inside spinal fluid 5, is indicated by a click produced by the different resistance to penetration of spinal fluid 5 and dura mater 6.

Once the click is produced, spindle 3 is withdrawn, parallel to axis A, from outer body 2, and the syringe is connected to the rear hole of outer body 2 to conduct the anaesthetic along outer body 2 and into spinal fluid 5 through opening 4.

Figures 2 to 8 show a needle 11 in accordance with the invention, which extends along an axis B, and comprises a tubular cylindrical outer body 12 for conducting anaesthetic into spinal fluid 5; a spindle 13 inserted hermetically inside outer body 12 and withdrawable, parallel to axis B, from outer body 12; and an inserter 14 coaxially surrounding outer body 12 and for assisting insertion and correct positioning of needle 11.

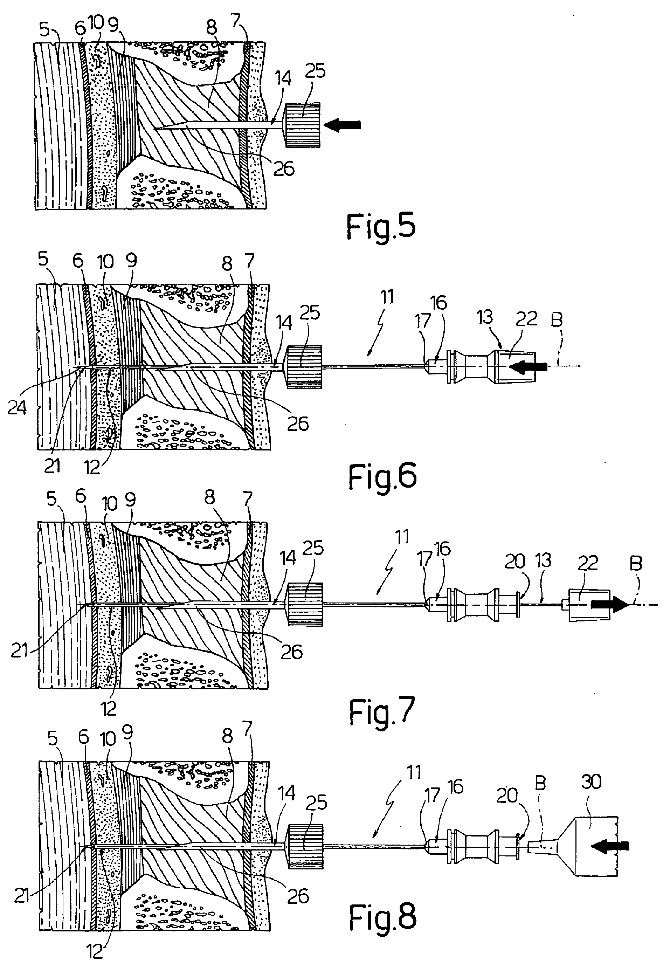

With particular reference to Figures 3 and 4, outer body 12 has a rear hole 15, which is sealed by spindle 13 when inserting needle 11, is opened when spindle 13 is withdrawn, and is then connected hydraulically to a syringe 30 (Figure 8) containing the anaesthetic.

Outer body 12 projects from a connecting portion 16 housing hole 15 and comprising a conical front end 17 by which to insert outer body 12 into inserter 14; and a rear end surface 20 defining an inlet 18 terminating in hole 15 and by which to insert spindle 13 inside outer body 12. Surface 20 extends perpendicularly to axis B, and comes to rest against spindle 13 to arrest insertion of spindle 13 into outer body 12.

Spindle 13 projects from a connecting portion 22 having a surface 23 perpendicular to axis B and which comes to rest against surface 20 of connecting portion 16 to arrest insertion of spindle 13 into outer body 12; and spindle 13 is withdrawn from outer body 12 by drawing connecting portion 22 away from connecting portion 16.

At the opposite end to hole 15, outer body 12 has a front hole 21, which is sealed by spindle 13 when inserting needle 11, and which injects anaesthetic into spinal fluid 5 once spindle 13 is withdrawn from outer body 12.

An important aspect of the present invention is that hole 21 is coaxial with axis B to more effectively inject the anaesthetic into spinal fluid 5.

More specifically, and with reference to Figures 3, 4 and 6, at the opposite end to connecting portion 22, spindle 13 has a conical-tipped end 24, which projects from hole 21 when spindle 13 is arrested inside outer out body 12, and disengages hole 21 when spindle 13 is withdrawn from outer body 12.

As shown in Figures 5 to 8, inserter 14 is cylindrical, is connectable to outer body 12 by means of a hollow body 25 defining an inlet for outer body 12, and, at the opposite end to hollow body 25, has an end 26 for insertion into the epidermis and sloping with respect to axis B.

In actual use, as shown in Figure 5, inserter 14 is inserted into the epidermis so that end 26 is inserted completely through superspinous ligament 7 and partly through interspinous ligament 8, and so that hollow body 25 projects from the epidermis.

The next step is to insert spindle 13 and outer body 12, which the operator is provided with in the Figure 3 configuration, in which spindle 13 is inserted to seal hole 15, and end 24 of spindle 13 projects from and seals front hole 21.

As shown in Figure 6, once inserted, spindle 13 and outer body 12 both project from end 26 of inserter 14.

More specifically, spindle 13 and outer body 12 are inserted so that they are housed inside inserter 14 along a portion extending completely through superspinous ligament 7 and partly through interspinous ligament 8, and project from inserter 14 along a portion extending through the rest of interspinous ligament 8 and completely through yellow ligament 9, peridural space 10, and dura mater 6, so that hole 21 and end 24 project inside spinal fluid 5.

Spindle 13 and outer body 12 are inserted in a direction coincident with axis B, and correct positioning of hole 21 and end 24 inside spinal fluid 5 and through to the other side of dura mater 6 is indicated by a click produced by hole 21 and end 24 piercing dura mater 6.

At this point, as shown in Figure 7, spindle 13 is withdrawn from outer body 12 to extract end 24 from spinal fluid 5 and open rear hole 15.

Syringe 30 (Figure 8) can now be connected to rear hole 15 through inlet 18 to feed the anaesthetic into outer body 12 and inject the anaesthetic into spinal fluid 5 through hole 21 in outer body 12.

The advantages of the present invention will be clear from the foregoing description.

In particular, the operating space inside spinal fluid 5 of both needle 11 and spindle 13 is less than a third of that of known needles, as shown in Figures 1 to 3, thus reducing the risk and consequences of trauma to the spinal cord.

Needle 11 provides for effectively injecting the anaesthetic into spinal fluid 5.

That is, hole 21 being coaxial with axis B, the click is produced when hole 21 is completely inside spinal fluid 5. As a result, all the anaesthetic is injected into spinal fluid 5, with no risk of part of it being dispersed inside dura mater 6.

Also on account of hole 21 being coaxial with axis B, needle 11 injects the anaesthetic evenly into spinal fluid 5, in each radial direction with respect to axis B.

Given the small size of hole 21 parallel to the insertion direction of needle 11, needle 11 also provides for injecting anaesthetic into regions, such as the cervical region, in which the subarachnoid space is small crosswise to the insertion direction of needle 11.

Finally, needle 11 also permits troublefree insertion of catheters, e.g. for prolonged spinal anaesthesia, by hole 21 being coaxial with axis B of outer body 12.

Clearly, changes may be made to spinal anaesthesia needle 11 as described and illustrated herein without, however, departing from the scope of the present invention, as defined in the accompanying Claims.

In particular, hole 21 may be formed with various degrees of roughness.

## Claims

1. A needle (11) for spinal anaesthesia, comprising an outer body (12) for conducting anaesthetic into a patient's body through an opening (21); and a spindle (13) selectively movable inside said outer body (12) to seal/unseal said opening (21);
**characterized in that** said opening (21) is coaxial with an axis (B) of said needle (11).

2. A needle as claimed in Claim 1, **characterized in that** one end (24) of said spindle (13) defines a retractable tip of said needle.

3. A needle as claimed in Claim 2, **characterized in that** said end (24) of said spindle (13) engages said opening (21) and projects from said outer body (12) to prevent conduction of said anaesthetic, and is extracted from said outer body (12) to permit conduction of said anaesthetic.
